**Europäisches Patentamt**

**European Patent Office**

**Office européen des brevets**

(19)

(11) Veröffentlichungsnummer : **0 180 038**
**B1**

(12) **EUROPÄISCHE PATENTSCHRIFT**

(45) Veröffentlichungstag der Patentschrift :
27.07.88

(51) Int. Cl.⁴ : **A 61 M 16/00, A 61 M 16/20**

(21) Anmeldenummer : 85112225.9

(22) Anmeldetag : 26.09.85

(54) **Luftimpulserzeuger bei Beatmungsgeräten.**

(30) Priorität : 29.09.84 DE 3435849

(43) Veröffentlichungstag der Anmeldung :
07.05.86 Patentblatt 86/19

(45) Bekanntmachung des Hinweises auf die Patenterteilung : 27.07.88 Patentblatt 88/30

(84) Benannte Vertragsstaaten :
CH DE FR GB LI

(56) Entgegenhaltungen :
EP-A- 0 075 542
DE-A- 2 831 312
DE-A- 2 831 313
DE-A- 2 834 037
DE-A- 3 212 097
US-A- 4 106 503
US-A- 4 155 356
US-A- 4 171 697

(73) Patentinhaber : Firma Carl Freudenberg
Höhnerweg 4
D-6940 Weinheim/Bergstrasse (DE)

(72) Erfinder : Stroh, Norbert
Schillerstrasse 50
D-7037 Magstadt (DE)
Erfinder : Lunkenheimer, Paul Peter, Prof. Dr. med.
Robert-Koch-Strasse 48
D-4400 Münster (DE)

(74) Vertreter : Weissenfeld-Richters, Helga, Dr.
Höhnerweg 2
D-6940 Weinheim/Bergstrasse (DE)

**Beschreibung**

Die Erfindung betrifft eine Vorrichtung gemäß dem ersten Teil von Anspruch 1.

Eine Einführung in dieses Gebiet mit weiteren Literaturstellen gibt die « Medizintechnik Produktinformation » der Firma Drägerwerk AG, unter dem Titel « Hochfrequenzbeatmung » oder « Health Equipment Information » Dezember 1983 — Nr. 119, mit der bibliographischen Kennung ISSN 0261-0736, beschrieben.

Nachteilig an den bekannten Vorrichtungen ist :

Der Luftimpulserzeuger befindet sich weit entfernt von der Stelle, an der die Atmungsluft in die Luftröhre austritt.

Die Erfinder haben gefunden, daß die unterschiedlichen Arten (HFPV, HFO, HFJV usw.) am besten arbeiten, wenn die zur Frequenz gehörigen Impulse scharfkantig sind, d. h. möglichst steile Anstiegflanken und möglichst steile Abfallflanken haben. Ausgehend von dieser Erkenntnis haben die bekannten Vorrichtungen folgende Nachteile :

a) Der Hochfrequenzerzeuger, der der Atemluft die Frequenz gibt, hat vom Auslass für die Beatmungsluft eine Entfernung, die grössenordnungsmässig im Meterbereich liegt. Allein schon durch die Laufzeit vom Erzeuger bis zum Auslass verschleifen die Impulse.

b) Die Beatmungsluftleitung hat einen Durchmesser im Bereich von 1-2 mm soweit es sich um die Zufuhrleitung handelt. Diese Zufuhrleitung wird ja mit ihrem Ende in einen Beatmungstubus eingeklebt und bläst in eine Leitung, die in der Wand des Tubus vorgesehen ist, die also schmäler sein muss, als die Wandstärke des Tubus. Eine solche Leitung bestimmt dann den effektiven Widerstand für die Luft. Im Stand der Technik ist eine solche Leitung des Tubus z. B. 0,7 mm breit und 1 mm hoch. Auch durch diese hohen Luftwiderstände wird der Impuls verschliffen. Dabei ist noch nicht einmal gesagt, dass diese im Tubus verlaufende Leitung die engste Stelle ist. Vielmehr kann man nicht dafür garantieren, dass der Klebstoff am Übergang von Tubusleitung zur Zufuhrleitung den dortigen Beatmungsluft-Leitungsquerschnitt nicht nochmals verengt.

c) Alle diese Leitungen verlaufen in Kunststoff, der die für diese Zwecke bekannte Weichheit hat. Gegenüber Impulsen weichen diese auf manchen Strecken nicht einmal einen Zehntelmillimeter dicken Leitungen aus und verschleifen damit die Impulse noch weiter.

d) Die angewendeten Frequenzen der Hochfrequenzbeatmung liegen bei 1 bis 50 Hz. Je hochfrequenter das System wird, desto mehr verschleifen die Impulse als Funktion der Frequenz. Für manche Anwendungsfälle sind jedoch die Frequenzen um so interessanter, je höher sie sind.

Der Stand der Technik erlaubt es gar nicht, höhere Frequenzen anzuwenden, denn es bringt nichts, im Atemluft-Frequenzerzeuger hohe Frequenzen zu erzeugen, wenn am Auslass praktisch nur noch ein Gleichstrom ankommt.

e) In einer Leitung bestimmt der engste Querschnitt den Strömungswiderstand. Im Stand der Technik gibt es die oben erwähnten sehr dünnen Leitungen, die einen hohen Strömungswiderstand haben. Trotzdem muss man genügend Beatmungsluft am Auslass zur Verfügung stellen. Dies erreicht man nicht anders als durch die bis an die Grenze getriebene Erhöhung des Drucks der Beatmungsluft. Damit tritt die Luft am Auslass mit extrem hoher Geschwindigkeit aus und trifft dann natürlich irgendwo mit hohem Impuls auf die Luftröhre oder Stammbronchien. Diese werden an der Auftreffstelle punktuell belastet, weil dort der Oberflächenschleim weggeblasen wird, weil dort die Wärmebilanz nicht mehr stimmt, weil dort die Feuchtgkeitsbilanz nicht mehr stimmt usw.

Aufgabe der Erfindung ist es, das Verschleifen der Impulse auf einfache Weise zu vermeiden.

Erfindungsgemäß wird dies durch die aus dem kennzeichnenden Teil des Hauptanspruches ersichtlichen Merkmale erreicht.

Man kann nun die Beatmungsluft im Gleichstrom heranführen. Der Beatmungslufterzeuger braucht also keine Druckpulsation mehr zu erzeugen und wird deshalb billiger. Der Strömungsimpuls steht mit steilen Flanken am Auslaß der Beatmungsluftleitung zur Verfügung und ist dadurch von besonders großer Wirksamkeit. Er ermöglicht es beispielsweise, bestimmte Teile einer Lunge in Resonanz kommen zu lassen, was ganz neuartige Effekte auf dem Gebiet der Hochfrequenz-Beatmung ermöglicht.

Ein weiterer Vorteil eines derartigen Gerätes besteht darin, daß grundsätzlich eine von der Kompliance der Lunge unabhängige Beatmung erreicht werden kann. Die alveoläre Ventilation eines Lungensegmentes hängt nur noch ab von der Frequenz und der vorgegebenen Abstrahlungs-charakteristik des Strömungsimpulses.

Durch die Merkmale des Anspruchs 2 kann man sehr klein bauende Ventile realisieren.

Durch die Merkmale des Anspruchs 3 kann man ein ruhig liegendes Steuerkabel verwenden, denn bei einem mechanischen Ventil muss sich das Steuerkabel ja bewegen.

Durch die Merkmale des Anspruchs 4 braucht das (mechanische) Steuerkabel sich lediglich zu drehen, was für diese Anwendung eine einfachere Betätigung mit sich bringt als z. B. bei einem hin- und hergehenden Steuerkabel, das zwar auf Zug ganz gut beanspruchbar ist, bei Druck jedoch gerne ausweicht.

Durch die Merkmale des Anspruchs 5 erreicht man, daß das Ventil in axialer Richtung kurz baut und dementsprechend die Biegsamkeit des Auslasses nicht zu sehr von der Ventil-Konstruktion beeinflußt wird. Ein Flachschieberventil hat kurze Durchlässe, so daß auch hier die Impulse steilflankig werden. Außerdem hat ein Flachschieberventil wenig rotatorische Masse, so daß Unwuchten von vornherein nicht ins Gewicht fallen. Wenn der Rotor des Flachschieberventils wenig Masse hat, dann bedeutet dies auch, daß er sofort einer Drehgeschwindigkeitsänderung des mechanischen Steuerkabels folgt.

An sich könnte ein Steuerkabel in Form einer biegsamen Welle auch am Aussenumfang des Rotors angreifen. Dann benötigt man jedoch eine Untersetzung zwischen dem Umfang des Rotors und desjenigen Zahnrads, das durch die biegsame Welle angetrieben wird. Dies vermeidet man durch die Merkmale des Anspruchs 6. Man erhält dann einen direkten Antrieb des Rotors.

Durch die Merkmale des Anspruchs 7 kann man die seither bekannten Tuben unverändert weiterverwenden und setzt in diese nur die erfindungsgemässe Vorrichtung ein. Sogar während der Behandlung kann von der erfindungsgemässen Hochfrequenzbeatmung auf die seitherige bestimmungsgemässe Verwendung eines Tubus übergegangen werden, indem man die erfindungsgemässe Vorrichtung einfach aus dem Tubus herauszieht. Die biegsame Welle, die Beatmungsluftleitung und das Ventil bilden dann eine Baueinheit, die für sich herstellbar, lagerbar und verwendbar ist.

Durch die Merkmale des Anspruchs 8 kann die Beatmungsluft in grossen Querschnitten zurückfliessen, obwohl für die Zuführung der Beatmungsluft ebenfalls grosse Querschnitte zur Verfügung stehen.

Durch die Merkmale des Anspruchs 9 kann man die Impulshöhe aber auch die Impussteilheit weiterverbessern, indem man dort einen negativen Strömungswiderstand vorsieht.

Eine Lösung gemäss den Merkmalen des Anspruchs 10 erfordert lediglich rotatorische Zuführung der Energie, was einfacher ist als eine hin- und hergehende Zuführung.

An sich könnte man zwei Wellen vorsehen, von denen die eine das Ventil steuert und die andere die Luftschaufelvorrichtung antreibt. Verfährt man jedoch gemäss dem Anspruch 11, so dient die biegsame Welle nicht nur als Steuerorgan, sondern auch als Energiezuführungsorgan.

Durch die Merkmale des Anspruchs 12 benötigt man keine zusätzliche, separate Luftschaufelvorrichtung.

Durch die Merkmale des Anspruchs 13 kommt man zu hohen Frequenzen, obwohl die Welle sich mit einer Drehzahl dreht, die nur ein Teil dieser Frequenzen ist.

Durch die Merkmale des Anspruchs 14 gelangen die scharfkantigen Impulse nicht nur an die Verzweigungsstelle der Stammbronchien, sondern sogar noch etwas in die Stammbronchien hinein. Man ist dadurch sicher, dass die Impulsfront nicht auf die Innenwand der Luftröhre oder der Stammbronchien trifft, sondern dass der Impuls in die Stammbronchien hinein fortgepflanzt wird.

Durch die Merkmale des Anspruchs 15 erreicht man, dass der Rotor an keiner Stelle der Luftröhre oder Stammbronchien anliegen kann und dadurch in seiner Rotation gebremst würde und man vermeidet auch, dass der Rotor Verletzungen durch seine Rotation verursachen könnte.

Durch die Merkmale des Anspruchs 16 erreicht man, daß diejenigen Öffnungen, aus denen die Impulse austreten, mindestens ungefähr in die zwei Stammbronchien zielen.

Mit einem Beatmungsgerät in der beschriebenen Form unter Berücksichtigung der Ansprüche 1 bis 16 ist eine Beatmung mit positivem Mitteldruck möglich. Durch den sich bei der Beatmung einstellenden Druck strömt die Ausatemluft in dem zwischen Beatmungsgerät und Tubus bestehenden Ringspalt ins Freie. Der Nachteil der Betriebsweise mit Überdruck, der sich abhängig von der benötigten Luftmenge einstellt und bei hohem Luftbedarf physiologisch nicht mehr tragbare Werte erreichen würde, kann durch eine Saugleitung kompensiert werden. Diese Saugleitung wird außerhalb des Mundes an den Ringspalt zwischen Tubus und Beatmungsgerät angeschlossen und die herausströmende Atemluft kann aktiv und definiert abgesaugt werden. Über die Höhe des Soges und Menge der zugeführten Luft kann der Atemmitteldruck eingestellt werden.

Derartige Beatmungsgeräte in kleiner Bauform, um kleine Luftstromwiderstände zu erhalten, eignen sich vorzugsweise für eine Aerosol- und Puderinsufflation. Das die Luftimpulse erzeugende Ventil am lungenwärts gerichteten Ende des Beatmungsgerätes eignet sich in vorzüglicher Weise als Zerstäuber. Eine Aerosolinsufflation eignet sich für eine gegebenenfalls streng lokalisierbare Lungenparenchymmobilisierung, d. h. einer Mukusmobilisation. Eine Puderinsufflation dient der Bronchografie, wobei der Puder als Kontrastmittel benutzt wird. Auch eine Gerinnungsaktivierung ist bei entsprechender Benutzung erreichbar.

Das Ventil ist zweckmäßig mit zwei Austrittsöffnungen für die Atemluft versehen, welche in einer gemeinsamen Ebene mit der Achse der Beatmungsluftleitung liegen und mit deren gedachter Verlängerung einen Winkel von etwa 30° einschließen. Die Zuordnung entspricht in etwa derjenigen, die die Stammbronchien in bezug auf die Luftröhre einnehmen und gewährleistet so eine verbesserte Beatmung. Eine relative Verdrehung muß dabei selbstverständlich vermieden werden, weshalb es sich als zweckmäßig erwiesen hat, die Lage der Austrittsöffnung durch eine sich über die gesamte Länge der Beatmungsluftleitung und/oder des Tubus erstreckende Markierung zu kennzeichnen.

Das Ventil kann neben der Austrittsöffnung mit wenigstens einer Gegenblasöffnung versehen werden, welche in Richtung der lungenseitig offenen Mündung der Rückluftleitung geöffnet ist. Beim Austreten eines scharfen Luftstrahles aus der Gegenblasöffnung ergibt sich so in der Rückluftleitung eine Art Venturi-Effekt, wodurch es gelingt, verbrauchte Atemluft aus der Lunge zu entfernen. Der diesbezügliche Effekt kommt vor allem dann sehr wirkungsvoll zum Tragen, wenn die Austrittsöffnungen und die Gegenblasöffnungen des Ventils zeitlich verschoben und insbesondere alternierend geöffnet werden. Eine gemeinsame Öffnungseinrichtung ist daher von Vorteil.

Die Austrittsöffnung und/oder die Gegenblasöffnung können mit einer Drosselvorrichtung versehen werden, wobei es sich als zweckmäßig erwiesen hat, wenn die Drosselvorrichtung so beschaffen ist, daß sich durch ihre Betätigung das Verhältnis der die Austrittsöffnungen und die Gegenblasöffnungen passierenden Luftströme in gegenseitiger Abhängigkeit verstellen läßt. Die spezifischen Verhältnisse während der Druck und der Saugphase lassen sich hierdurch leicht an individuelle Belange des Patienten anpassen.

Der Tubus und die Beatmungsluftleitung können fest verbunden bzw. einstückig ausgebildet sein, was die Gewährleistung optimaler Strömungsbedingungen im Bereich des Ventils erleichtert. Auch eine mittige Zuordnung des Profils der Beatmungsluftleitung zu demjenigen des Tubus ist diesbezüglich von Vorteil.

Eine außermittige Zuordnung des Profils der Beatmungsluftleitung zu demjenigen des Tubus führt demgegenüber zu einer vereinfachten Biegbarkeit in einer Richtung. Das sachgerechte Einlegen der Vorrichtung in die Luftröhre wird hierdurch erleichtert.

Der Tubus und die Beatmungsluftleitung können auch von einander unabhängige Teile bilden, was es gestattet, zunächst den Tubus in die Luftröhre einzulegen und anschließend in denselben die Beatmungsluftleitung einzuführen. Die Anbringung und die Entfernung ist in diesem Falle sehr erleichtert und gestattet zusätzlich die Erzielung eines gleichmäßigen Überganges von der erfindungsgemäßen Druck-Sogbeatmung des Patienten über die bekannte künstliche Beatmung unter Verwendung pulsierender Überdrücke zu der natürlichen Atmung. Kritische Genesungsphasen lassen sich hierdurch weitgehend vermeiden.

Die Ausblasöffnungen sollen der Tubuswandung in einer möglichst präzise definierten Weise zugeordnet sein, um eine gute Sogwirkung zu erhalten. Die Anwendung von Führungsmitteln hat sich unter diesem Gesichtspunkt als vorteilhaft bewährt.

Die erfindungsgemäße Vorrichtung gestattet eine künstliche, intensive Beatmung mit einer Beatmungsfrequenz von 3/min. bis 150/sec., somit des gesamten in Frage kommenden Frequenzbereichs. Die Vorrichtung ist einfach anzubringen und behindert in keiner Weise eine während der Behandlung wiederkehrende Spontanatmung. Sekretbildungen in der Lunge lassen sich wesentlich besser beherrschen als bisher.

Die Erfindung wird anhand von Ausführungsbeispielen beschrieben. In der Zeichnung zeigen :

Fig. 1 eine Vorderansicht des unteren Bereiches einer Luftröhre, der Stammbronchien und der rechten und der linken Lunge,

Fig. 2 eine systematische Darstellung des Volumens der Lunge,

Fig. 3 eine systematische Seitenansicht der Erfindung,

Fig. 4 ein Schnitt längs der Linie 4 in Fig. 3,

Fig. 5 einen Längsschnitt durch den Endbereich der erfindungsgemäßen Vorrichtung,

Fig. 6 eine Ansicht gemäß dem Pfeil A in Fig. 5,

Fig. 7 eine schematische Ansicht aus dem Bereich der Luftröhre, der Stammbronchien und mit der Lage des Verteilers.

Fig. 8 einen Längsschnitt durch den Endbereich einer anderen Ausführung der erfindungsgemäßen Vorrichtung,

Fig. 9 die in Figur 8 gezeigte Ausführung der erfindungsgemäßen Vorrichtung in einer 90° geschwenkten Schnittebene.

Figuren 10 bis 13 die Endbereiche weiterer Varianten der erfindungsgemäßen Vorrichtung. Das Ventil (89) enthält bei der Ausführung nach Figur 10 einen mechanisch angetriebenen Drehschieber, bei der Ausführung nach Fig. 11/12 einen mechanisch angetriebenen Stellschieber und bei der Ausführung nach Figur 13 einen elektromagnetisch angetriebenen Stellschieber.

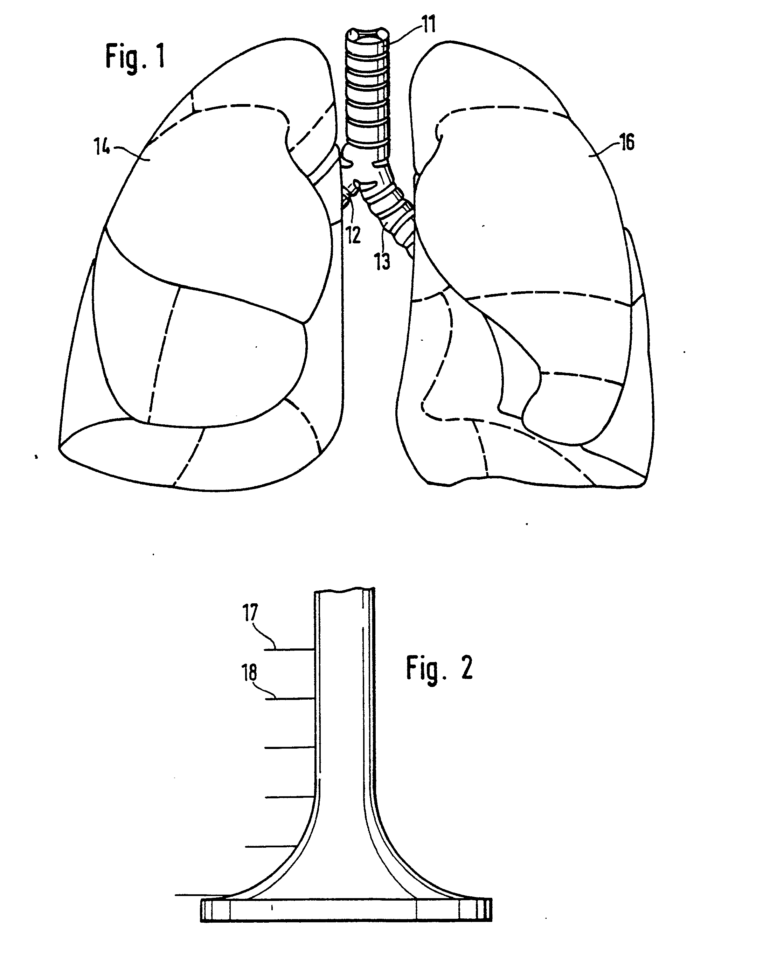

In Figur 1 sieht man eine Luftröhre 11 und 2 Stammbronchien 12, 13. Wie man sieht zweigt die rechte Stammbronchie 12 unter einem stumpferen Winkel ab als die Stammbronchie 13 und ist auch ein wenig kürzer. Die Stammbronchien 12, 13 führen in die rechte Lunge 14 und die linke Lunge 16. Die Figur 1 zeigt unterschiedliche Lungenlappen, die in bekannter Weise die jeweiligen Lungen bilden. Es handelt sich hier um Einzelteile dieses Organs, die recht unterschiedlich auf Frequenzen ansprechen. Insbesondere haben sie unterschiedliche Resonanzfrequenzen, abhängig vom Alter des Individuums, abhängig von der Größe, abhängig von evtl. Kankheiten, abhängig von evtl. Verletzungen usw.

Wenn der eine Lungenteil in oder nahe einer Resonanz ist, dann kann der andere trotzdem weit ausserhalb der Resonanz sein. Es ist deshalb notwendig, dass man die Frequenz der Impulse erhöhen und erniedrigen kann, sei es zu diagnostischen Zwecken, sei es zu therapeutischen Zwecken.

Die Bronchien verzweigen sich wiederholt (12 bis 15 mal), so dass man auch vom Bronchialbaum spricht. Das Lumen, das für den Luftstrom zur Verfügung steht, vergrössert sich von der Luftröhre bis zur Peripherie etwa exponentiell. Die Fig. 2 zeigt schematisch den Verlauf des Lumens. Der Strich 17 bedeutet den Verzweigungspunkt der Luftröhre in die Stammbronchien, der Strich 18 die weiteren Teilungsschritte peripheriewärts. Da die Beatmungsluft-Impulse etwa beim Strich 17 erzeugt werden, ist es wegen des prinzipiellen Verlaufs von Fig. 2 um so wichtiger, mindestens dort steilflankige Impulse zu erzeugen, denn nach innen zu ist der Aufbau der Lunge ohnehin geeignet, die Impulsflanken zu verschleifen.

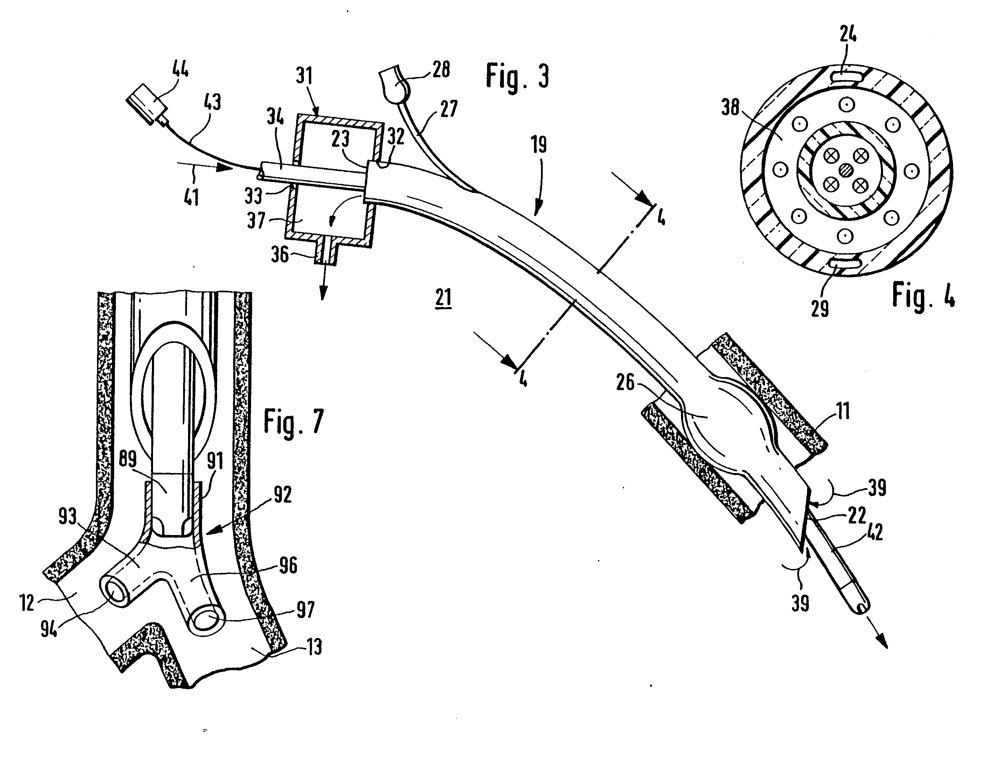

Gemäss Fig. 3 ist ein Tubus 19 bekannter Art vorgesehen, der in der mit der Zeichnungsebene zusammenfallende Ebene 21 gekrümmt ist. Er ist in üblicher Weise aus einem transparenten PVC-Schlauch gefertigt, der unten den zur besseren Einführung dienenden Schrägschnitt 22 hat, der oben einen Radialschnitt 23 hat, einen Aussendurchmesser von 11,5 mm besitzt, eine Wandstärke von etwa 1,4 mm hat, in seinem gemäss Fig. 4 oberen Wandbereich eine Leitung 24 besitzt, die unten mit einer aufblasbaren Abdichtmanschette 26 kommuniziert und in die oben eine Anschlussleitung 27 in üblicher Weise angeklebt ist, die in eine Aufsteckmanschette 28 übergeht, die auf einen Druckerzeuger aufgeschoben werden kann, mittels dessen Hilfe die Abdichtmanschette 26 so aufgeblasen werden kann, dass sie an der Innenwand der Luftröhre dichtend anliegen kann. Gegenüber der Leitung 24 ist eine weitere Leitung 29 üblicher Art vorgesehen, die als Absaugung für Sekret oder dergleichen dient.

Im Bereich des Radialschnitts 23 ist ein Fassungskopf 31 vorgesehen, der mit einer unteren Öffnung 32 den dortigen Bereich des Tubus 19 luftdicht fasst. Mit einer koaxial gegenüberliegenden Öffnung 33 fasst er luftdicht eine Beatmungsluft-Leitung 34. Über einen Stutzen 36 wird aus dem Hohlraum 37 und damit auch aus dem Raum 38 zwischen der Innenwand des Tubus 19 und der Leitung 34 verbrauchte Luft abgesaugt, die dort am Schrägschnitt 22 gemäss dem Pfeil 39 eindringt.

Die Beatmungsluft wird der Leitung 34 gemäss dem Pfeil 41 zugeführt. Die Beatmungsluft hat Gleichstrom-Charakter und es muss unter allen Umständen vermieden werden, dass der Druck der Beatmungsluft gegenüber dem Druck der Umgebungsluft negativ wird, weil dies zum sofortigen Lungenverschluss führen würde. Am besten ist es, wenn die Beatmungsluft einen Drucküberschuss von etlichen -zig Millibar gegenüber der Umgebungsluft hat. Ziel dieses positiven Druckunterschieds ist, dass die Lunge auf jeden Fall aufgebläht wird. Die Leitung 34 besteht aus einem Kunststoffschlauch. Sein Aussendurchmesser ist 7 mm und sein Innendurchmesser ist 5 mm. Er ist glasklar und aus PVC. Sein über den Fassungskopf 31 hinausragender Teil ist an einen Beatmungslufterzeuger in nicht dargestellter Weise angeschlossen. Wie Fig. 3 zeigt, ragt die Leitung 34 mit ihrem Endbereich 42 etwas nach unten aus dem Tubus 19 heraus. In der Leitung 34 liegt eine biegsame Welle 43 aus rostfreiem Stahl, die 1,2 mm Durchmesser hat. Sie ist homogen, d. h. kein gewickelter Draht und vermag so einerseits erhebliche Momente zu übertragen und ist andererseits so biegsam, dass sie der Gesamtkrümmung gemäss Fig. 3 folgt. Im Gegensatz zu spiralig gewickelten biegsamen Wellen braucht man hier auch nicht auf die Drehrichtung zu achten, denn gewickelte biegsame Wellen soll man mit einer Drehrichtung beaufschlagen, die die Festigkeit des Wickels erhöht. Ausserdem nimmt eine nicht gewickelte, homogene Welle wenig Platz weg, so dass zwischen der Innenwand 45, der Leitung 34 und der biegsamen Welle 43 ausserordentlich viel Querschnitt für die Beatmungsluft bleibt. Die Welle 43 wird von einem in seiner Drehzahl regelbaren Elektromotor 44 angetrieben. Sein Steuerungsbereich liegt zwischen 600 und 2 000 u/min. Der Motor 44 kann schon im Beatmungslufterzeuger eingebaut sein, so dass die biegsame Welle 43 schon von Anfang an in der Leitung 34 liegt. Oder aber kann die biegsame Welle 43 an einer weiter aussen liegenden, nicht gezeichneten Stelle mit einer luftdichten Durchführung durch die Wand 46 der Leitung 34 geführt sein.

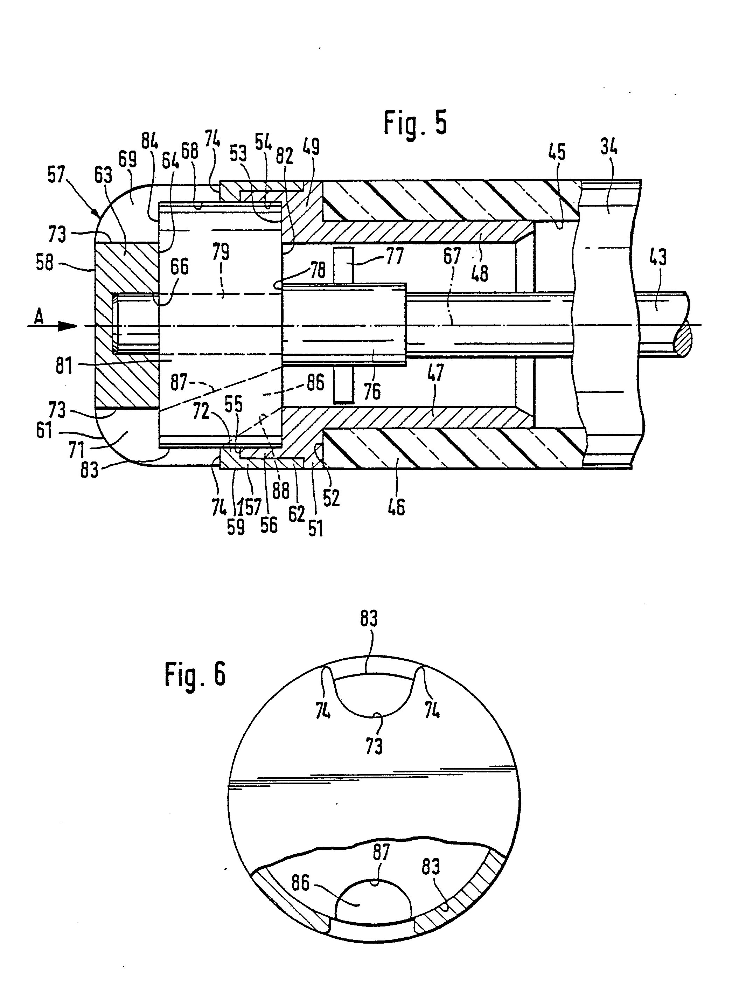

Am Endbereich 42 ist in die Leitung 34 ein Gehäuseflansch 47 eingeschoben, der ein koaxiales Rohrstück 48 aufweist, dessen Aussenfläche auf einem wenig grösseren Durchmesser als die Innenwand 45 liegt, so dass dort ein Preßsitz entsteht. Gemäss Fig. 5 ist links vom Rohrstück 48, ein im Innendurchmesser gleicher Bund 49 koaxial vorgesehen, der mit einer Kreisringrippe 51 dicht an der Stirnfläche 52 der Leitung 34 anliegt. Die Kreisringrippe 51 hat exakt den gleichen Aussendurchmesser, wie die anschliessende Leitung 34, so dass dort ein glatter, stufenloser, die Einführung in den Tubus 19 erleichternder Übergang entsteht. Der Bund 49 hat nach links eine koaxiale, exakt radiale Kreisringfläche 53, die nach links in eine koaxiale Kreiszylinderfläche 54 übergeht, so dass zwischen beiden ein rechter Winkel vorhanden ist. Die Kreiszylinderfläche 54 ist die Innenfläche einer nach links gerichteten, dünnen Fassungswand 56. Die Fassungswand 56 endet nach links mit einer schmalen koaxialen kreisringförmigen Stirnfläche 55.

Eine Kappe 57 ist ebenfalls koaxial und in ihrem Aussenumfang im wesentlichen rotationssymmetrisch. Zwischen ihrer linken Stirnfläche 58 und ihrer kreiszylindrischen Umfangsfläche 59 ist eine Abrundung 61 mit verhältnismässig grossem Radius vorgesehen. Die Kappe 57 übergreift mit einer koaxialen kreiszylindrischen, dünnen Wand 62 die Fassungswand 56 bis zur Kreisringrippe 51, mit deren Umfangsfläche sie fluchtet, so dass auch hier kein stufenförmiger Übergang vorhanden ist. Die Wand 157 der aus rostfreiem Stahl bestehenden Kappe 57 ist fest mit der Fassungswand 56 verbunden, sei es durch Verkleben oder Preßsitz. Rechts von der Stirnfläche 58 befindet sich ein relativ dicker Boden 63, in den von seiner Innenfläche 64 aus koaxial ein Sackloch 66 eingearbeitet ist. Die Innenfläche 64 ist koaxial und exakt radial zur geometrischen Längsachse 67. Senkrecht zur Innenfläche 64 steht koaxial eine kreiszylindrische Umfangsfläche 68, die sich stufenlos in der Kreiszylinderfläche 54 fortsetzt.

Jeweils um 180° gegeneinander versetzt und symmetrisch zur Ebene 21 sind in der Kappe 57 zwei Austrittslöcher 69, 71 vorgesehen, die teilweise im Boden 63 und teilweise in der Umfangswand 72 der Kappe 57 verlaufen. Die Austrittslöcher 69, 71 haben innere Scheitel 73 und äussere Scheitel 74, wobei die Scheitel 73 im Boden 63 und die Scheitel 74 in der Umfangswand 72 liegen.

Ein Terminal 76 fasst das gemäss Fig. 5 linke Ende der biegsamen Welle 43 drehfest. Auf dem koaxialen Terminal 76 sitzen Luftschaufeln 77, die Flügel einer solchen Winkellage haben, dass sie bei einer bestimmten Drehrichtung der biegsamen Welle 43 Beatmungsluft nach links drücken. Nach einer radialen Stufe 78 geht das Terminal 76 in einen kreiszylindrischen, koaxialen Zapfen 79 über, dessen linker Endbereich im Sackloch 66 drehbar geführt ist. Auf dem Zapfen 79 ist mit Preßsitz ein Rotor 81 drehfest aufgeschoben, dessen rechte Kreisringfläche 82 drehbar, jedoch ziemlich luftdicht an der Kreisringfläche 53 anliegt und dort auch — samt der Welle 43 — einen Anschlag für etwaige Bewegungen nach rechts findet. Der kreisringförmige Umfang 83 des Rotors 81 wird drehbar von der Umfangsfläche 68 und der mit ihr fluchtenden Kreiszylinderfläche 54 geführt, so dass über das Rotationsspiel hinausgehende Radialbewegungen nicht möglich sind und auch eine gewisse Luftdichtigkeit erzielt wird. Wie man sieht, ragt der Umfang 83 in die Austrittslöcher 69, 71 hinein, ohne jedoch über sie hinauszuragen. Parallel zur Kreisringfläche 82 ist links eine gleich grosse Kreisringfläche 84 vorgesehen, die ebenfalls teilweise in die Austrittslöcher 69, 71 ragt, sonst aber von der Innenfläche 64 geführt wird.

Im Rotor 81 ist eine spitzwinkelig nach aussen verlaufende Austrittsöffnung 86 vorgesehen. Wie der Fig. 5 entnehmbar ist, führt der innere Scheitel 87 vom Umfang des Terminal 76 zum Scheitel 73, so dass nach dieser Richtung hin eine maximal grosse Austrittsöffnung 86 entsteht. Nach der anderen Seite ist die Austrittsöffnung 86 deshalb maximal, weil der äussere Scheitel 88 vom Innenumfang des Bunds 49 bis zum Scheitel 74 verläuft. Damit öffnet sich die Austrittsöffnung 86 trichterförmig und ihre nach aussen geneigte Lage in Verbindung mit der Rotationsenergie zieht Beatmungsluft aktiv nach und macht nicht nur Impulse aus ihr.

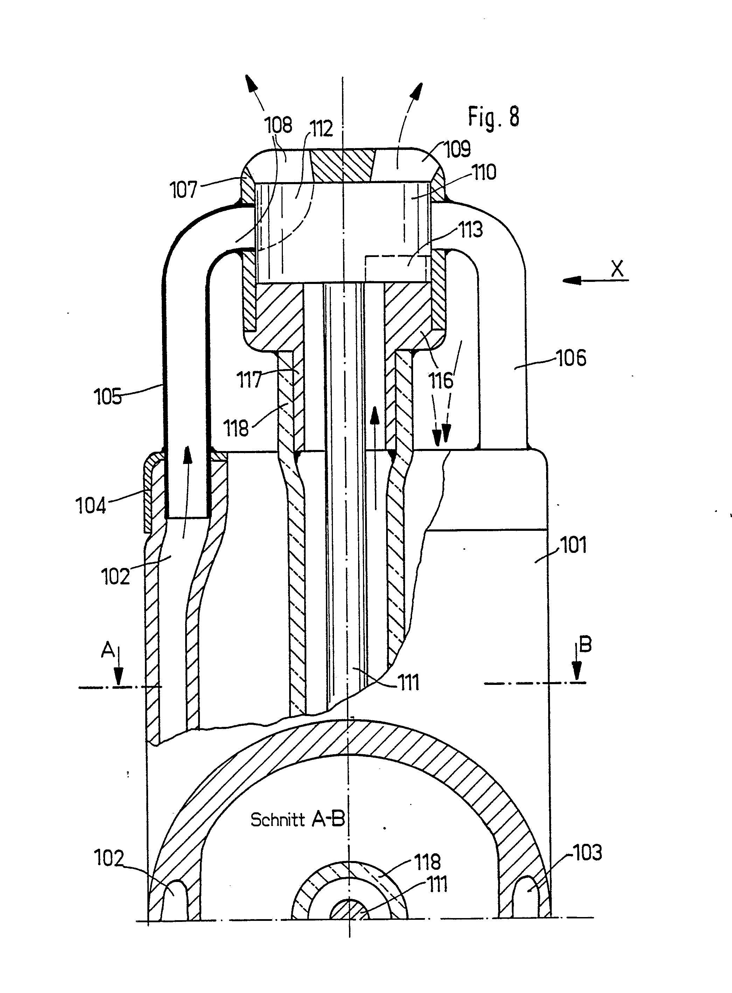

Gemäss Fig. 7 ist auf dieses Drehschieberventil 89 der Bund 91 einer Hose 92 geschoben, deren linkes Hosenbein 93 mit seiner Öffnung 94 in die Stammbronchie 12 zielt und gemäss dieser unter einem stumpferen Winkel verläuft, während das Hosenbein 96 mit seiner Öffnung 97 in die Stammbronchie 13 zielt und gemäss dieser etwas spitzwinkeliger verläuft. Gemäss Fig. 7 sind jedoch die Hosenbeine 93, 96 im Aussendurchmesser wesentlich kleiner als die Stammbronchien im Innendurchmesser, so dass weitaus genügend Raum für den Rückfluss verbrauchter Beatmungsluft übrig bleibt. Wie man aus Fig. 7 auch sieht, zielen die Austrittslöcher 69, 71 in die richtige Richtung, sofern der Bund 91 richtig aufgeschoben worden ist.

Beim Einführen der Beatmungsvorrichtung wird sich nach etwas gefühlvollem Vorschieben und Drehen das am Rotationskopf befindliche elastische Formteil anatomie- und damit funktionsgerecht einpassen. Dies zeigt zum einen, dass die Vorrichtung richtig sitzt, zum andern wird beim Betrieb eine unbeabsichtigte Verdrehung verhindert.

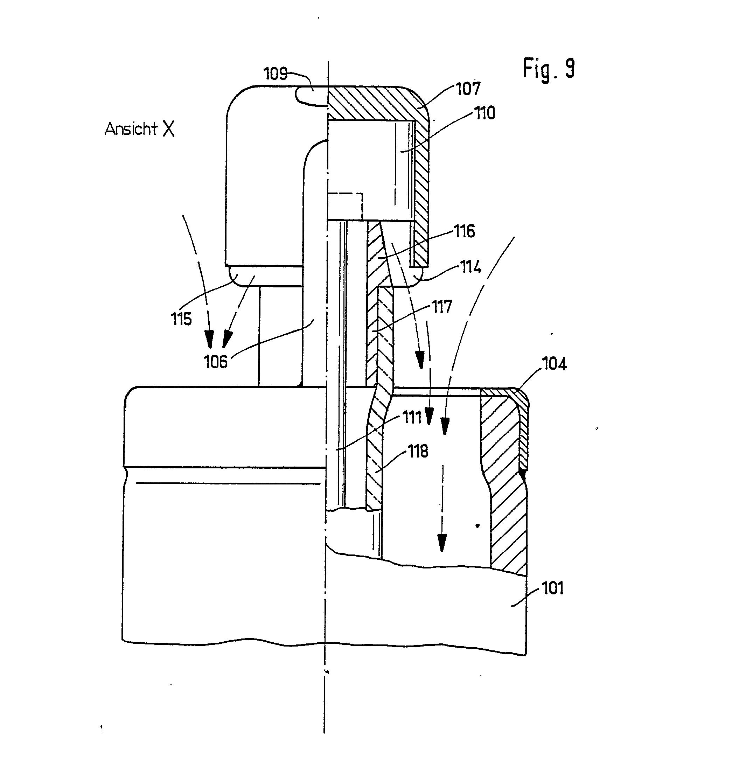

In den Figuren 8 und 9 ist ein Ausführungsbeispiel für ein oszillierenes Beatmungsgerät dargestellt. Ein bereits in den Figuren 3 und 4 beschriebener Tubus 101 ist dahingehend modifiziert, daß er neben den bereits erwähnten, in die Wandung eingelassenen Kanälen für die Sekretabsaugung und die Druckluftversorgung für eine Dichtmanschette zwei weitere Luftversorgungskanäle 102/103 enthält. Das Ende dieses Tubus wird von einem Ring 104 gefaßt, der aufgepreßt und mit dem Tubus verklebt ist. Der Ring ist so geformt, daß die Außenabmessungen des Tubus im wesentlichen erhalten bleiben.

Der Ring ist so auf den Tubus aufgesetzt, daß zwei dünnwandige, in den Ring eingelassene Rohre 105/106 in die im Tubus integrierten Luftversorgungskanäle 102/103 hineinragen. Diese Versorgungskanäle werden dabei aufgeweitet und somit ein fester und druckdichter Sitz erreicht. Zusätzlich werden auch diese Verbindungen verklebt.

An ihrem anderen Ende sind die Rohre 105, 106 um 90° gebogen und ragen in eine Kappe 107, mit der sie fest verbunden sind. In der Kappe sind symmetrisch zur Mittellinie um 180° gegeneinander versetzt zwei Austrittsöffnungen 108, 109 vorgesehen.

Im oberen Teil dieser Kappe ist ein Rotor 110 drehbar gelagert. Dieser Rotor ist fest mit einer flexiblen Welle 111 verbunden, über die er in eine Drehbewegung versetzt werden kann. In dem Rotor sind zwei Nuten eingefräst, wovon über die eine 112 die lungenwärts liegenden Austrittsöffnungen 108, 109, über die andere 113 die mundwärts, einen Sog in der Mündung des Ringes 104 erzeugenden Ausblasöffnungen 114, 115 angesteuert werden. Diese mundwärts gerichteten Ausblasöffnungen 114, 115 sind in einem Rundkörper 116 eingelassen, der in den unteren Teil der Kappe hineinragt mit dieser fest verbunden ist und zusammen mit der Kappe das Lager für den Rotor bildet. Der Rundkörper 116 besitzt eine rohrförmige Verlängerung 117, über die ein zentral im Tubus liegender Schlauch 118 geschoben ist. Der Schlauch wird dabei aufgeweitet und es ergibt sich eine druckfeste Verbindung, die darüber hinaus zusätzlich verklebt ist. Der Schlauch 118 hat zwei Funktionen ; zum einen dient er als Führung für die flexible Welle 111, zum anderen als Luftzuführungsleitung für die mundwärts geöffneten Gegenblasöffnungen 114, 115. Die aus den Austrittsöffnungen 108, 109 austretende Luftströmung läßt sich dadurch unabhängig von der aus den Gegenblasöffnungen 114, 115 austretenden Luftströmung von außen verändern.

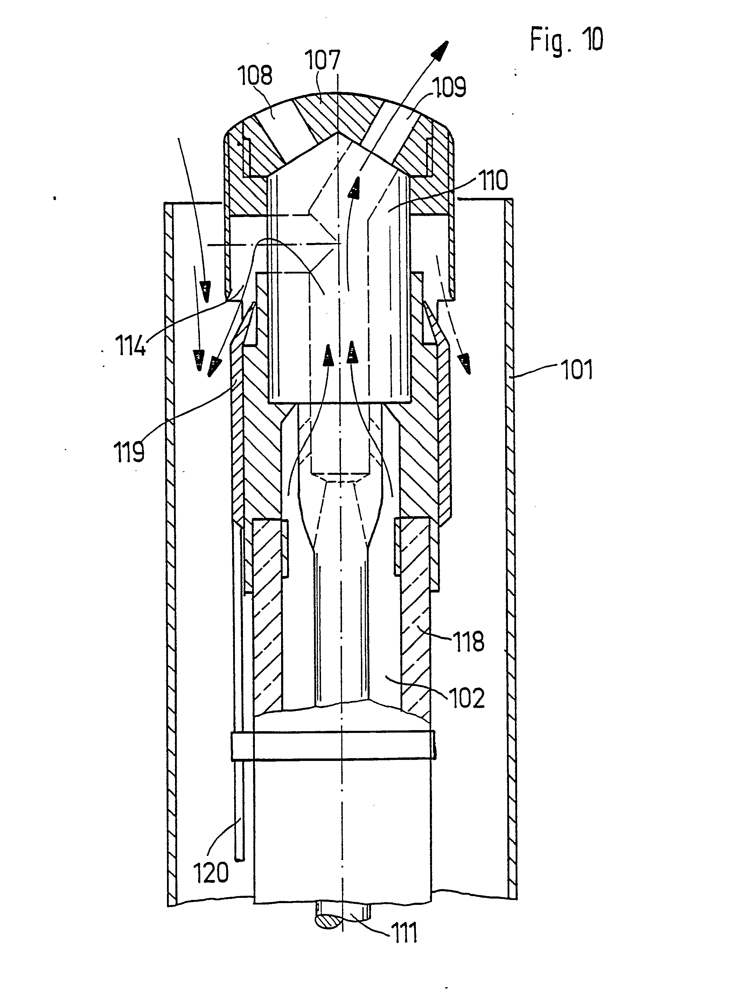

Die Ausführung nach Figur 10 ist der vorstehend beschriebenen funktionell ähnlich. Das Ventil ist auch in diesem Falle als Drehschieberventil ausgebildet, wobei der Rotor 110 durch die biegsame Welle 111 angetrieben ist. Im Gegensatz zu der vorstehend beschriebenen Ausführung ist in diesem Falle jedoch nur eine einzige Versorgungleitung 102 vorgesehen, die durch den Raum zwischen der flexiblen Welle 111 und der diese mit Abstand umgebenden Schlauchleitung 118 gebildet wird. Diese bildet zugleich die Beatmungsluftleitung und ist am oberen Ende an dem Stator 107 des Gehäuses festgelegt. In diesem ist der Rotor 110 relativ verdrehbar gelagert. Der Innenraum des Rotors steht durch seitliche Öffnungen mit dem Innraum der Beatmungsluft-

leitung in einer ständigen Verbindung und die durch die Beatmungsluftleitung zugeführte Druckluft wird je nach Stellung des Rotors aus dessen Innenraum entweder über die Austrittsöffnungen 108, 109 in Richtung der Lunge oder über die durch einen Ringspalt gebildete Gegenblasöffnung 114 in Richtung des Mundes in den Tubus 101 geleitet. Hierbei wird zugleich verbrauchte Atemluft aus den Hohlräumen der Lunge abgesogen und durch den Tubus ins Freie geführt.

Die Gegenblasöffnung 114 wird durch einen Ringspalt gebildet, dessen Durchströmquerschnitt durch axiale Verschiebung des Stellkegels 119 veränderbar ist. Eine entsprechende axiale Verschiebung des Stellkegels 119 kann von außen mittels der Verstelleinrichtung 120 erfolgen. Das Verhältnis zwischen dem je Atmungstakt eingespeisten und abgesogenen Luftvolumens läßt sich dadurch beliebig einstellen, wobei die große Funktionssicherheit von hervorzuhebendem Vorteil ist.

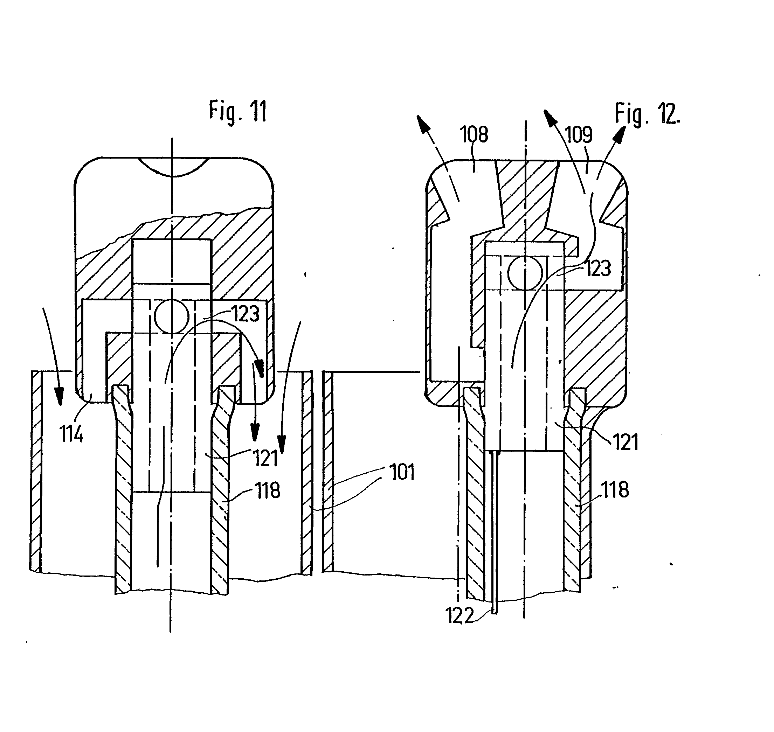

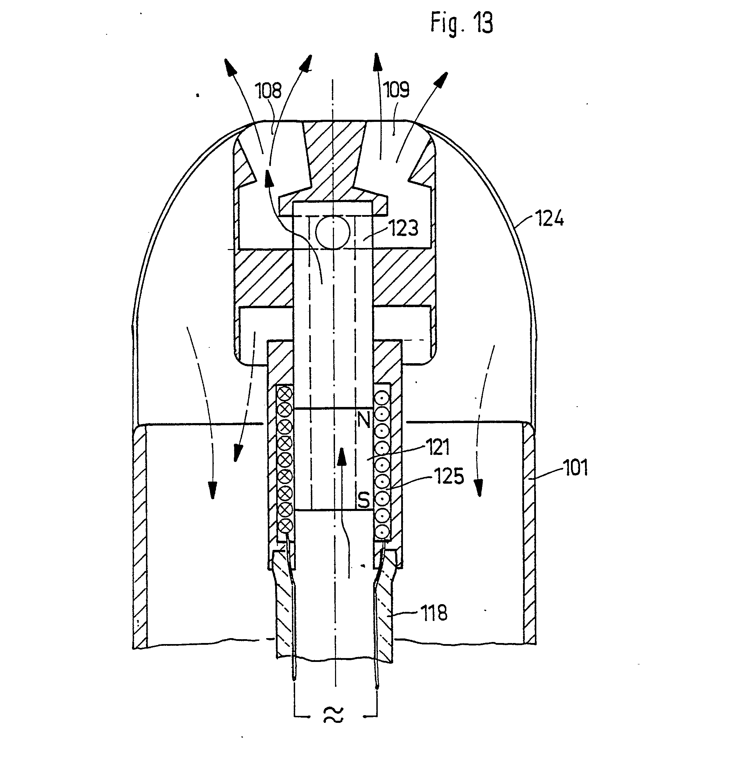

Figur 11 und 12 nehmen Bezug auf eine vereinfachte Ausführung, bei der ein Flachschieberventil zur Anwendung gelangt. Der in der oben geschlossenen Beatmungsluftleitung 118 gelagerte Stellkörper 121 ist durch eine Verstelleinrichtung 122 axial verschiebbar, wobei die Durchtrittsöffnung 123 abwechselnd mit einer den Austrittsöffnungen 108, 109 zugeordneten bzw. einer der Ausblasöffnung 114 zugeordneten Bohrung in Deckung gebracht wird. Das durch die Beatmungsluftleitung 118 zugeführte Luftvolumen wird dadurch alternierend als frische Atemluft in die Lunge gelenkt bzw. zur Absaugung verbrauchter Atemluft in den Innenraum des Tubus 101. Sie gelangt durch denselben dann ins Freie. Die außermittige Zuordnung der Beatmungsluftleitung 118 zu dem Tubus 101 wird durch Fig. 12 verdeutlicht.

Die in Figur 13 gezeigte Ausführung ist funktionell der vorstehend beschriebenen ähnlich. Für die Verstellung des Flachschiebers ist in diesem Falle ein elektromagnetischer Antrieb vorgesehen, und die enthaltenen Teile sind organisch zu einer Einheit verbunden. Der Tubus wird stirnseitig durch ein gewölbtes Korbgebilde 124 begrenzt, wodurch das Einlegen in die Luftröhre erleichtert wird.

Der zur Anwendung gelangende Flachschieber besteht aus einem magnetisierten Werkstoff und ist zur Vermeidung galvanischer oder elektrischer Wechselwirkungen mit einer Oberflächenbeschichtung aus nichtleitendem Kunststoff versehen. Er ist in der Führung der Beatmungsluftleitung 118 durch die Wirkung der elektromagnetischen Kräfte der Spule 125 axial verschiebbar, wobei seine Durchtrittsöffnung die aus der Beatmungsluftleitung zugeführte Frischluft in Abhängigkeit von der Richtung des Magnetfeldes abwechselnd in Richtung der Austrittsöffnungen 108, 109 lenkt bzw. in Richtung der Gegenblasöffnung 114. Im erstgenannten Falle wird Frischluft in die Lunge eingespeist, im letztgenannten Falle unter Ausnutzung von Injektorwirkungen verbrauchte Atemluft aus derselben abgesogen. Die dabei erzielbaren Schaltfrequenzen sind erheblich und lassen therapeutische Anwendungen in weitem Rahmen zu. Erschütterungen werden vom Patienten weitestgehend ferngehalten, und das Einlegen in die Luftröhre gestaltet sich bei der Verwendung flexibler Leiter für die Zuführung des elektrischen Stromes zumeist einfacher als bei den vorstehend beschriebenen Varianten. Die hohe Ansprechgenauigkeit ist von weiterem, hervorzuhebendem Vorteil.

**Patentansprüche**

1. Vorrichtung zur Erzeugung von Luft-Impulsen bei Beatmungsgeräten, die mit Hochfrequenzbeatmung arbeiten, mit einem Ventil (89), mit einer Beatmungsluftleitung (34), in deren Weg das Ventil (89) liegt, mit einem die Beatmungsluftleitung umgebenden flexiblen Tubus (19), an dessen lungenseitigem Endbereich sich ein Auslaß der Beatmungsluftleitung (34) für die Beatmungsluft befindet, dadurch gekennzeichnet, daß sich das Ventil (89) am lungenseitigen Ende der Beatmungsluftleitung (34) befindet und daß eine Steuereinrichtung (43, 111) zur Veränderung der Impuls-Frequenz des Ventils zum Ventil führt.

2. Vorrichtung nach Anspruch 1, dadurch gekennzeichnet, daß das Ventil (89) ein mechanisches Ventil ist.

3. Vorrichtung nach Anspruch 1, dadurch gekennzeichnet, daß das Ventil (89) ein elektrisches Ventil ist.

4. Vorrichtung nach Anspruch 1 und 2, dadurch gekennzeichnet, daß das Ventil (89) ein Drehschieberventil mit einem Rotor (81) und einem Stator (57) ist.

5. Vorrichtung nach Anspruch 4, dadurch gekennzeichnet, daß das Ventil (89) ein Flachschieberventil ist.

6. Vorrichtung nach Anspruch 4 oder 5, dadurch gekennzeichnet, daß zum Drehschieberventil eine antreibbare, biegsame Welle (43) führt, deren dortiges Ende koaxial mit dem Rotor (81) des Drehschieberventils verbunden ist.

7. Vorrichtung nach Anspruch 2 oder 3, dadurch gekennzeichnet, daß die Beatmungsluftleitung (34) getrennt vom Ventil (89) in den Tubus einführbar ist.

8. Vorrichtung nach Anspruch 7, dadurch gekennzeichnet, daß der Raum zwischen der inneren Tubuswand und der Beatmungsluftleitung (34) die Rückluftleitung bildet.

9. Vorrichtung nach Anspruch 2 oder 3, dadurch gekennzeichnet, daß unmittelbar vor dem Ventil (89) eine Beatmungsluft-Druckerhöhungsvorrichtung vorgesehen ist.

10. Vorrichtung nach Anspruch 9, dadurch gekennzeichnet, daß die Druckerhöhungsvorrichtung eine Luftschaufelvorrichtung ist.

11. Vorrichtung nach Anspruch 9, dadurch gekennzeichnet, daß die Druckerhöhungsvorrichtung von der einzigen biegsamen Welle (111) angetrieben wird.

12. Vorrichtung nach Anspruch 4, dadurch gekennzeichnet, daß das Drehschieberventil zugleich als Schaufelrad ausgebildet ist.

13. Vorrichtung nach Anspruch 4, dadurch gekennzeichnet, daß der Rotor (81) und/oder der Stator (57) wenigstens eine Öffnung hat.

14. Vorrichtung nach Anspruch 1, dadurch gekennzeichnet, daß stromab auf das Ventil (89) ein rohrförmiger Verteiler aus extrem weichem Silikongummi aufgeschoben ist, der zwei unter spitzem Winkel laufende Äste (93, 96) entsprechend dem Stammbronchienverlauf aufweist, und daß der Außendurchmesser der Äste wesentlich kleiner als der Innendurchmesser der jeweiligen Stammbronchien ist.

15. Vorrichtung nach Anspruch 4, dadurch gekennzeichnet, daß der Rotor (110) vom Stator (107) umschlossen wird und nirgends über den Umfang des Stators (107) hinausragt.

16. Vorrichtung nach Anspruch 13, dadurch gekennzeichnet, daß der Stator (107) zwei Austrittsöffnungen (108, 109) um 180° gegeneinander versetzt hat und daß die Austrittsöffnungen in der Krümmungsebene des Tubus (101) liegen.

17. Vorrichtung nach Anspruch 16, dadurch gekennzeichnet, daß die Lage der Austrittsöffnungen (108, 109, 69, 71) durch eine sich über die gesamte Länge der Beatmungsluftleitung (34) und/oder des Tubus (101) erstreckende Markierung gekennzeichnet ist.

18. Vorrichtung nach einem der Ansprüche 1 bis 17, dadurch gekennzeichnet, daß das Ventil (89) neben der Austrittsöffnung für die Beatmungsluft mit wenigstens einer Gegenblasöffnung (114) versehen ist und daß die Gegenblasöffnung (114) in Richtung der offenen Mündung der Rückluftleitung geöffnet ist.

19. Vorrichtung nach Anspruch 18, dadurch gekennzeichnet, daß die Austrittsöffnung (69, 71 ; 108, 109) und die Gegenblasöffnung (114) oder mehrere Austrittsöffnungen (69, 71 ; 108, 109) und Gegenblasöffnungen (114) in Beziehung zueinander zeitlich verschoben durchströmbar sind.

20. Vorrichtung nach Anspruch 18 oder 19, dadurch gekennzeichnet, daß die Austrittsöffnung (108, 109) und/oder die Gegenblasöffnung (114) mit einer Drosselvorrichtung versehen ist.

21. Vorrichtung nach einem der Ansprüche 18 bis 20, dadurch gekennzeichnet, daß durch die Drosselvorrichtung das Verhältnis der durch die Austrittsöffnung und durch die Gegenblasöffnung durchtretenden Luftströmungen veränderbar ist.

22. Vorrichtung nach einem der Ansprüche 1 bis 21, dadurch gekennzeichnet, die Beatmungsluftleitung (34) und der Tubus (101) fest verbunden sind.

23. Vorrichtung nach Anspruch 22, dadurch gekennzeichnet, daß die Beatmungsluftleitung (34) dem Profil des Tubus (101) mittig zugeordnet ist.

24. Vorrichtung nach Anspruch 22, dadurch gekennzeichnet, daß die Beatmungsluftleitung (34) dem Profil des Tubus (101) außermittig zugeordnet ist.

25. Vorrichtung nach einem der Ansprüche 22 bis 24, dadurch gekennzeichnet, daß die Beatmungsluftleitung (34) durch wenigstens einen in der Wandung des Tubus (101) ausgesparten Hohlraum (102, 103) gebildet wird.

## Claims

1. A device for generating an air pulse in a respirator operating with high-frequency respiration, having a valve (89), a respiration-air line (34), in thepath of which the valve (89) is located, and a flexible tube (19) surrounding the respiration-air line, an outlet of the respiration-air line (34) for the respiration air being provided in the end region of the flexible tube on the same side as the lungs, characterised in that the valve (89) is arranged at the end of the respiration-air line (34) on the same side as the lungs, and in that a control means (43, 111) for varying the pulse frequency of the valve leads to the valve.

2. A device according to claim 1, characterised in that the valve (89) is a mechanical valve.

3. A device according to claim 1, characterised in that the valve (89) is an electrical valve.

4. A device according to claim 1 and 2, characterised in that the valve (89) is a rotary slide valve with a rotor (81) and a stator (57).

5. A device according to claim 4, characterised in that the valve (89) is a flat slide valve.

6. A device according to claim 4 or 5, characterised in that there leads to the rotary slide valve a drivable flexible shaft (43), of which the end located there is connected coaxially to the rotor (81) of the rotary slide valve.

7. A device according to claim 2 or 3, characterised in that the respiration-air line can be introduced into the tube separately from the valve (89).

8. A device according to claim 7, characterised in that the space between the inner tube wall and the respiration-air line (34) forms the return-air line.

9. A device according to claim 2 or 3, characterised in that a respiration-air pressure-increasing device is provided immediately in front of the valve (89).

10. A device according to claim 9, characterised in that the pressure-increasing device is an air-scoop device.

11. A device according to claim 9, characterised in that the pressure-increasing device is driven by the single flexible shaft (111).

12. A device according to claim 4, characterised in that the rotary slide valve is designed at the same time as a scoop wheel.

13. A device according to claim 4, characterised in that the rotor (81) and/or the stator (57) have at least one orifice.

14. A device according to claim 1, characterised in that downstream of the valve (89) a tubular distributor which is made of extremely soft silicone rubber and which has two branches (93, 96) extending at an acute angle and corresponding to the form of the main bronchia is pushed on, and in that the outside diameter of the branches is significantly less than the inside diameter of the respective main bronchia.

15. A device according to claim 4, characterised in that the rotor (110) is surrounded by the stator (107) and nowhere projects beyond the periphery of the stator (107).

16. A device according to claim 13, characterised in that the stator (107) has two outflow orifices (108, 109) offset 180° relative to one another, and in that the outflow orifices are in the plane of curvature of the tube (101).

17. A device according to claim 16, characterised in that the position of the outflow orifices (108, 109, 69, 71) is defined by a marking extending over the entire length of the respiration-air line (34) and/or of the tube (101).

18. A device according to any one of claims 1 to 17, characterised in that the valve (89) is provided with at least one counterblowing orifice (114) next to the outflow orifice for the respiration air, and in that the counterblowing orifice (114) is opened in the direction of the open mouth of the return-air line.

19. A device according to claim 18, characterised in that the outflow orifice (69, 71 ; 108, 109) and the counterblowing orifice (114) or the several outflow orifices (69, 71 ; 108, 109) and counterblowing orifices (114) are arranged such that flow can pass through them in a manner staggered in time in relation to one another.

20. A device according to claim 18 or 19, characterised in that the outflow orifice (108, 109) and/or the counterblowing orifice (114) are provided with a throttling device.

21. A device according to any one of claims 18 to 20, characterised in that the ratio of the air flows passing through the outflow orifice and through the counterblowing orifice can be varied by means of the throttling device.

22. A device according to any one of claims 1 to 21, characterised in that the respiration-air line (34) and the tube (101) are firmly connected.

23. A device according to claim 22, characterised in that the respiration-air line (34) is coordinated with the profile of the tube (101) in a central manner.

24. A device according to claim 22, characterised in that the respiration-air line (34) is coordinated with the profile of the tube (101) in an off-centre manner.

25. A device according to any one of claims 22 to 24, characterised in that the respiration-air line (34) is formed by at least one cavity (102, 103) made in the wall of the tube (101).

**Revendications**

1. Dispositif pour engendrer des impulsions d'air pour appareil de respiration mettant en œuvre une respiration à haute fréquence et comprenant une soupape (89), une conduite d'air de respiration (34) dans le parcours de laquelle est disposée la soupape (89), un tube flexible (19) entourant la conduite d'air de respiration à l'extrémité pulmonaire de laquelle se trouve une sortie pour l'air de respiration de la conduite d'air de respiration (34), caractérisé en ce que la soupape (89) est prévue à l'extrémité pulmonaire de la conduite d'air de respiration (34) et en ce qu'un dispositif de commande (43, 111) pour la modification de la fréquence des impulsions de la soupape est relié à celle-ci.

2. Dispositif selon la revendication 1, caractérisé en ce que la soupape (89) est une soupape mécanique.

3. Dispositif selon la revendication 1, caractérisé en ce que la soupape (89) est une soupape électrique.

4. Dispositif selon les revendications 1 ou 2, caractérisé en ce que la soupape (89) est une soupape à tiroir rotatif avec un rotor (81) et un stator (57).

5. Dispositif selon la revendication 4, caractérisé en ce que la soupape (89) est une soupape à tiroir coulissant plat.

6. Dispositif selon la revendication 4, caractérisé en ce qu'un câble flexible (43) mène à la soupape à tiroir rotatif, et dont l'extrémité coaxiale correspondante est reliée au rotor (81) de la soupape à tiroir rotatif.

7. Dispositif selon les revendications 2 ou 3, caractérisé en ce que la conduite d'air de respiration (34) peut être introduite indépendamment de la soupape (89) dans le tube.

8. Dispositif selon la revendication 7, caractérisé en ce que l'espace entre la paroi intérieure du tube et la conduite d'air de respiration (34) forme la conduite de retour d'air.

9. Dispositif selon les revendications 2 ou 3, caractérisé en ce qu'un dispositif de pressurisation de l'air de respiration est prévu juste devant la soupape (89).

10. Dispositif selon la revendication 9, caractérisé en ce que le dispositif de pressurisation est un dispositif à ailettes de ventilation.

11. Dispositif selon la revendication 9, caractérisé en ce que le dispositif de pressurisation est actionné par le seul câble flexible (111).

12. Dispositif selon la revendication 4, caractérisé en ce que la soupape à tiroir rotatif est conçue, en même temps, comme roue à ailettes.

13. Dispositif selon la revendication 4, caractérisé en ce que le rotor (81) et/ou le stator (57) comporte(nt) au moins une ouverture.

14. Dispositif selon la revendication 1, caractérisé en ce qu'un distributeur tubulaire en caoutchouc silicone extrêmement mou comprenant deux branches (93, 96), s'étendant sous un angle aigu, conformément à l'allure des bronches, est engagé sur la soupape (89), du côté aval, et en ce que le diamètre extérieur des branches est sensiblement plus petit que le diamètre intérieur des bronches correspondantes.

15. Dispositif selon la revendication 4, caractérisé en ce que le rotor (110) est entouré par le stator (107) et ne dépasse nulle part la périphérie du stator (107).

16. Dispositif selon la revendication 13, caractérisé en ce que le stator (107) comporte deux ouvertures de sortie (108, 109), décalées angulairement de 180° l'une par rapport à l'autre et en ce que les ouvertures de sortie se trouvent dans le plan de courbure du tube (101).

17. Dispositif selon la revendication 16, caractérisé en ce que la position des ouvertures de sortie (108, 109, 69, 71) est marquée par un repère s'étendant sur toute la longueur de la conduite d'air de respiration (34) et/ou du tube (101).

18. Dispositif selon l'une quelconque des revendications 1 à 10, caractérisé en ce que la soupape (89) comporte au moins un orifice à contre-souffle (114) prévu à côté de l'ouverture de sortie de l'air de respiration et en ce que l'orifice à contre-souffle (114) est ouvert en direction de l'embouchure ouverte de la conduite de retour d'air.

19. Dispositif selon la revendication 18, caractérisé en ce que l'ouverture de sortie (69, 71, 108, 109) et l'orifice à contre-souffle (114) ou les ouvertures de sortie (69, 71, 108, 109) et les orifices à contre-souffle (114) sont traversés à des moments chronologiquement décalés.

20. Dispositif selon les revendications 18 ou 19, caractérisé en ce que l'ouverture de sortie (108, 109) et/ou l'orifice à contre-souffle (114) sont équipés d'un dispositif d'étranglement.

21. Dispositif selon l'une quelconque des revendications 18 à 20, caractérisé en ce que le rapport des circulations traversant l'ouverture de sortie et l'orifice à contre-souffle est modifiable à l'aide du dispositif d'étranglement.

22. Dispositif selon l'une quelconque des revendications 1 à 21, caractérisé en ce que la conduite d'air de respiration (34) et le tube (101) sont solidaires l'un de l'autre.

23. Dispositif selon la revendication 22, caractérisé en ce que la conduite d'air de respiration (34) est associée concentriquement au profil du tube (101).

24. Dispositif selon la revendication 22, caractérisé en ce que la conduite d'air de respiration (34) est associée excentriquement au profil du tube (101).

25. Dispositif selon l'une quelconque des revendications 22 à 24, caractérisé en ce que la conduite d'air de respiration (34) est formée par au moins un canal (102, 103) pratiqué dans la paroi du tube (101).

Fig. 1

Fig. 2

Fig. 3

Fig. 4

Fig. 7

0 180 038

**0 180 038**

Fig. 5

Fig. 6

3

Fig. 8

Schnitt A-B

Fig. 9

Ansicht X

Fig. 10

Fig. 11

Fig. 12.

108  109

123

123

114

121

121

118

118

101

122

0 180 038

Fig. 13